(19) Europäisches Patentamt / European Patent Office / Office européen des brevets

(11) EP 1 547 595 B1

# (12) EUROPEAN PATENT SPECIFICATION

(45) Date of publication and mention of the grant of the patent:
**08.10.2014 Bulletin 2014/41**

(21) Application number: **03766710.2**

(22) Date of filing: **01.08.2003**

(51) Int Cl.:
*A61K 31/42* (2006.01) *A61K 9/70* (2006.01)
*A61K 47/20* (2006.01) *A61K 47/32* (2006.01)
*A61K 47/34* (2006.01) *A61K 47/40* (2006.01)
*A61P 29/00* (2006.01) *A61P 43/00* (2006.01)

(86) International application number:
**PCT/JP2003/009830**

(87) International publication number:
**WO 2004/012734 (12.02.2004 Gazette 2004/07)**

## (54) PATCH FOR TRANSDERMAL ADMINISTRATION

PFLASTER ZUR TRANSDERMALEN VERABREICHUNG

PASTILLE SERVANT A EFFECTUER UNE ADMINISTRATION TRANSDERMIQUE

(84) Designated Contracting States:
**AT BE BG CH CY CZ DE DK EE ES FI FR GB GR HU IE IT LI LU MC NL PT RO SE SI SK TR**

(30) Priority: **02.08.2002 JP 2002226309**

(43) Date of publication of application:
**29.06.2005 Bulletin 2005/26**

(73) Proprietor: **HISAMITSU PHARMACEUTICAL CO., INC.**
**Tosu-shi,**
**Saga-ken 841-0017 (JP)**

(72) Inventors:
- **IKESUE, A.**
  **c/o Tsukuba Lab. of HISAMITSU PHARM. CO**
  **Tsukuba-shi, Ibaraki 305-0856 (JP)**
- **TATEISHI, N.**
  **c/o Tsukuba Lab. Pharm. Co. Inc.**
  **Tsukuba-shi, Ibaraki 305-08656 (JP)**
- **UEMURA, K.**
  **c/o Tsukuba Lab. of HISAMITSULABPHARM**
  **Tsukuba-shi, Ibaraki 305-0856 (JP)**
- **TERAHARA, T.**
  **c/o Tsukuba Lab. of HISAMITSU PHARM**
  **Tsukuba-shi, Ibaraki 305-0856 (JP)**
- **HIGO, N.c/o Tsukuba Laboratory of HISAMITSU PHARM**
  **Tsukuba-shi, Ibaraki 305-0856 (JP)**
- **SAKAI, M.**
  **c/o Tsukuba Lab. of HISAMITSU PHARM. CO.**
  **Tsukuba-shi, Ibaraki 305-0856 (JP)**

(74) Representative: **Grünecker, Kinkeldey, Stockmair & Schwanhäusser**
**Leopoldstrasse 4**
**80802 München (DE)**

(56) References cited:
**EP-A1- 1 170 004**
**WO-A1-02/02111**
**WO-A1-02/17923**
**WO-A1-2004/047815**
**WO-A1-2004/047816**
**WO-A2-02/096435**
**JP-A- 10 231 248**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## Technical Field

The present invention relates to a patch for transdermal administration comprising a backing and an adhesive layer comprising an adhesive base agent and a drug.

## Background Art

Conventionally, concerning an administration method of drug, the oral administration method using tablets, capsules, syrup or the like has been known. However, in these years, an approach is tried in which these drugs are administrated through skin by using a patch. Since the administration method using a patch has such advantages as decrease of administration frequency, improved compliance, easiness of administration and discontinuation, in addition to solutions of problems in the oral administration method, it is expected as a useful administration method of a drug in treatment for, especially, aged persons and children.

However, since a stratum corneum of normal skin has a barrier function of inhibiting exogenous materials from invading the body, a base agent used in ordinary patches often does not give sufficient transdermal permeation of compounded pharmaceutical ingredients. Further, since a stratum corneum has high lipid solubility, generally permeability of a drug is extremely low. Accordingly, upon administrating a drug through skin by using a patch, it is necessary to enhance a transdermal permeation property of a drug through a stratum corneum of skin.

On the other hand, among nonsteroidal antiinflammatory agents for use in the treatment of rheumatic diseases, arthritis, arthropathic and degenerative arthritic disorder, pain and the like, cyclooxygenase-2 (COX-2) inhibitors have been recently developed as a medicinal group that blocks selectively COX-2, thereby reducing risk of digestive adverse effect, and WO 02/02111 discloses a skin treatment system comprising 3-phenyl-4-(4-methylsulfonylphenyl)-2(5H)-furanone (generic name: rofecoxib) or 4-[5-(4-methylphenyl)-3-(trifluoromethyl)pyrazole-1-yl]benzolsulfonamide (generic name: celecoxib).

However, even in the case of rofecoxib or celecoxib described in the aforementioned gazette, the transdermal permeation property is not sufficient. Therefore, a patch for transdermal administration having a sufficiently high transdermal permeation property of a COX-2 inhibitor to exert a superior antiinflammatory effect has not been obtained yet.

## Disclosure of Invention

The present invention was achieved in consideration of the problems included in the aforementioned conventional technique, and the object of the present invention is to provide a patch for transdermal administration having a sufficiently high transdermal permeation property of a COX-2 inhibitor to exert a superior antiinflammatory effect.

The present inventors made efforts to achieve the aforementioned object, and found that 4-(5-methyl-3-phenylisoxazole-4-yl)benzenesulfonamide (generic name: valdecoxib) is a compound functioning as a COX-2 inhibitor with a sufficiently high transdermal permeation property, and that use of such valdecoxib or a pharmaceutically acceptable salt thereof enables us to obtain a patch for transdermal administration that exerts a superior antiinflammatory effect, thereby accomplishing the present invention.

Specifically, the patch for transdermal administration of the present invention is characterized in comprising a backing and an adhesive layer being placed on at least one side of the backing and comprising an adhesive base agent and a drug, wherein the drug is valdecoxib or a pharmaceutically acceptable salt thereof.

In the patch for transdermal administration of the present invention, the adhesive base agent preferably comprises at least one selected from the group consisting of styrene-isoprene-styrene copolymer, acrylic polymer and polyisobutylene, the adhesive base agent more preferably comprises styrene-isoprene-styrene copolymer and polyisobutylene.

Further, in the patch for transdermal administration of the present invention, more preferably the adhesive layer further comprises, in addition to the adhesive base agent and the drug, at least one kind selected from the group consisting of 1) polyhydric alcohol (polyethylene glycol is especially preferably), 2) cyclodextrin and derivatives thereof, and 3) pirotiodecane.

## Brief Description of Drawings

Fig. 1 is a graph showing the analgesic action of valdecoxib as a COX-2 inhibitor on yeast-induced paw inflammation and nociception in rats.

Fig. 2 is a graph showing the antiinflammatory effect of valdecoxib as a COX-2 inhibitor on carrageenin-induced paw edema in rats.

Fig. 3 is a graph showing the antiinflammatory effect of an acrylate patch comprising valdecoxib on carrageenin-induced paw edema in rats.

Fig. 4 is a graph showing the antiinflammatory effect of an SIS-based patch comprising valdecoxib on carrageenin-induced paw edema in rats.

**Best mode for carrying out the Invention**

Hereinafter, detailed explanation will be given about preferable embodiments of the patch for transdermal administration of the present invention.

The patch for transdermal administration of the present invention is characterized in comprising a backing and an adhesive layer being placed on at least one side of the backing and comprising an adhesive base agent and a drug, wherein the drug is valdecoxib or a pharmaceutically acceptable salt thereof.

As the backing for use in the patch of the present invention, any one may be used without particular limitation insofar as it can support the adhesive layer, and a stretchable or an unstretchable backing may be used. Specific examples of the backing include cloth, nonwoven cloth, polyurethane, polyester, polyvinyl acetate, polyvinylidene chloride, polyethylene, polyethylene terephthalate and aluminum sheet, one made of complex material thereof, and the like.

Thickness of the backing according to the present invention is preferably, but not particularly limited, in a range of from 5 to 1000 μm. A thickness of the backing lower than the lowest limit described above tends to decrease operation easiness upon sticking the patch and, on the other hand, that higher than the highest limit described above tends to decrease production easiness due to difficulty of cutting the backing or the patch, or the like in the production process of the patch.

In the patch of the present invention, an adhesive layer comprising an adhesive base agent and a drug is placed on the backing described above. Here, the adhesive layer according to the present invention is an adhesive layer that comprises valdecoxib or a pharmaceutically acceptable salt thereof, which is a cyclooxygenase-2 inhibitor, as the drug described above.

Valdecoxib used as a drug in the present invention is a compound 4-(5-methyl-3-phenylisoxazole-4-yl)benzenesulfonamide represented by the following structural formula (1)

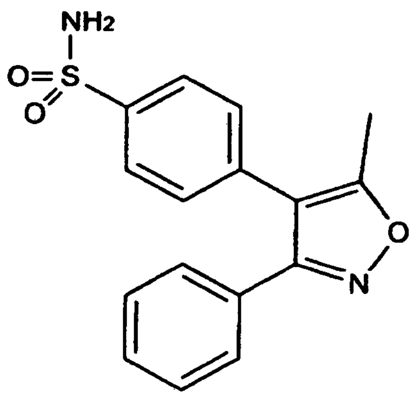

and the fact that it functions as a COX-2 inhibitor is described in the following document: (J. J. Talley et a1, 4-[5-Methyl-3-phenylisoxazol-4-yl]-benzenesulfonamide, Valdecoxib: A Potent and Selective Inhibitor of COX-2, J. Med. Chem., 43, 775-777, 2000). Valdecoxib for use in the present invention may be a free form or a pharmaceutically acceptable salt. Such pharmaceutically acceptable salts are preferably, but not limited specifically, sodium salt, potassium salt and ammonium salt, among them, ammonium salt is more preferable.

Compounding amount of the drug according to the present invention is not particularly limited, but preferably it is 0.1-50 weight % on the basis of the total amount of compounds contained in the adhesive layer. A compounding amount of the drug lower than the lowest limit described above tends to give an insufficient antiinflammatory effect provided by the drug and, on the other hand, that higher than the highest limit described above tends to decrease physical properties of the formulation.

In the patch for transdermal administration of the present invention, the adhesive layer comprises an adhesive base agent in addition to the drug. Here, as the adhesive base agent according to the present invention, rubber polymer and acrylic polymer can be mentioned, and the rubber polymer and acrylic polymer may be contained at the same time.

Examples of such rubber polymer include styrene-isoprene-styrene copolymer, polyisobutylene, isoprene rubber, styrene-butadiene-styrene copolymer, styrene-butadiene rubber and silicone rubber. Among them, use of styrene-isoprene-styrene block copolymer and/or polyisobutylene is more preferable because it tends to enhance both of skin permeability of the drug and physical properties of the formulation. These rubber polymers may be used individually by one kind or in combination of two or more kinds. Among them, use of styrene-isoprene-styrene block copolymer and polyisobutylene in combination is especially preferable because it tends to more enhance skin permeability of the drug and physical properties of the formulation.

As acrylic polymer, polymers employing alkyl (meth)acrylate esters in which carbon number in the alkyl group is 4 or more and, preferably, 15 or less, are preferable. Specific examples of such alkyl (meth)acrylate ester include alkyl (meth)acrylate esters having a linear alkyl group, a branched alkyl group such as butyl, pentyl, hexyl, heptyl, octyl, nonyl, decyl, undecyl, dodecyl and tridecyl. One kind or two or more kinds of these may be used.

Further, a copolymerizable monomer may be copolymerized in the aforementioned alkyl (meth)acrylate ester, if necessary. Examples of the monomer include carboxyl group containing monomers such as (meth)acrylic acid, itaconic acid, maleic acid or maleic anhydride; sulfoxyl group containing monomers such as styrenesulfonate, allylsulfonate, sulfopropyl(meth)acrylate, (meth)acryloyloxynaphthalenesulfonic acid or acrylamide methylpropanesulfonic acid; hydroxyl group containing monomers such as hydroxyethyl (meth)acrylate ester or hydroxypropyl (meth)acrylate ester; amide group containing monomers such as (meth)acrylamide, dimethyl(meth)acrylamide, N-butylacrylamide, N-methylol(meth)acrylamide or N-methylolpropane(meth)acrylamide; alkylaminoalkyl group containing monomers such as aminoethyl (meth)acrylate ester, dimethylaminoethyl (meth)acrylate ester or tert-butylaminoethyl (meth)acrylate ester; alkoxyalkyl (meth)acrylate esters such as methoxyethyl (meth)acrylate ester or ethoxyethyl (meth)acrylate ester; an alkoxy group (or ether bond in a side chain) containing (meth)acrylic esters such as tetrahydrofurfuryl (meth)acrylate ester, methoxyethylene glycol (meth)acrylate ester, methoxydiethylene glycol (meth)acrylate ester, methoxypolyethylene glycol (meth)acrylate ester or methoxypolypropylene glycol (meth)acrylate ester; and vinyl-series monomers such as (meth)acrylonitrile, vinyl acetate, vinyl propionate, N-vinyl-2-pyrrolidone, methylvinyl pyrrolidone, vinyl pyridine, vinyl piperidone, vinyl pyrimidine, vinyl piperazine, vinyl pyrazine, vinyl pyrrole, vinyl imidazole, vinyl caprolactam, vinyl oxazole and vinyl morpholine. One kind of or two kinds or more in combination of these may be copolymerized. These copolymerizable monomers can be used for the purpose of adjusting cohesion force of the adhesive layer or enhancing solubility of the drug. The copolymerization amount may be arbitrarily set within a range of 2-40 % by weight in accordance with purposes.

Further, as acrylic polymer, although there is no particular limitation only when it is a polymer copolymerized while incorporating at least one kind of (meth)acrylic acid derivatives represented by 2-ethylhexyl acrylate, methyl acrylate, butyl acrylate, hydroxyethyl acrylate, 2-ethylhexyl methacrylate and the like, for example, adhesives listed and described as an adhesive in Iyakuhin Tenkabutsu Jiten (Encyclopedia of Pharmaceutical Excipients) 2000 (edited by Japan Pharmaceutical Excipients Council) such as acrylic acid - octyl acrylate ester copolymer, 2-ethylhexyl acrylate - vinyl pyrrolidone copolymer solution, acrylic ester - vinyl acetate copolymer, 2-ethylhexyl acrylate - 2-ethylhexyl methacrylate - dodecyl methacrylate terpolymer, methyl acrylate - 2-ethylhexyl acrylate copolymer resin emulsion, adhesives such as acrylic polymer incorporated in an acrylic resin alkanolamine liquid, DURO-TAK acrylic adhesive series (manufactured by National Starch and Chemical), SK-DYNE MatriDerm (Soken Chemical and Engineering), Eudragit series (HIGUCHI SHOKAI) and the like may be usable.

Compounding amount of the adhesive base agent according to the present invention is not particularly limited but preferably it is 10-90 % by weight on the basis of the total amount of compounds contained in the adhesive layer. A compounding amount of the adhesive base agent lower than the lowest limit described above tends to give an insufficient adhesiveness of the adhesive layer and, on the other hand, that higher than the highest limit described above tends to give an insufficient permeability of the drug. Further, when styrene-isoprene-styrene block copolymer and polyisobutylene are used in combination as the adhesive base agent, compounding amount of the former of 7-63 % by weight and compounding amount of the latter of 3-27 % by weight on the basis of the total amount of compounds contained in the adhesive layer tend to enhance both of skin permeability of the drug and physical properties of the formulation, and so the instance is especially preferable.

In the patch for transdermal administration of the present invention, the adhesive layer may further comprise a permeation enhancer in addition to the drug and the adhesive base agent described above. As the permeation enhancer according to the present invention, compounds that are conventionally recognized to have an permeation enhancing effect on the skin may be used, including specifically fatty acids having 6 to 20 carbon atoms, aliphatic alcohols, fatty acid esters, fatty acid amides, and fatty acid ethers, aromatic organic acids, aromatic alcohols, aromatic organic acid esters and ethers, which may be saturated or unsaturated, and also may be linear, branched or cyclic. Further, in the present invention, lactic acid esters, acetic acid esters, monoterpenes, sesquiterpenes, Azone, Azone derivatives, glycerin fatty acid esters, propylene glycol fatty acid esters, sorbitan fatty acid esters (Span), polysorbates (Tween), polyethylene glycol fatty acid esters, polyoxyethylene hydrogenated castor oils (HCO), polyoxyethylene alkyl ethers, sucrose fatty acid esters or vegetable oils may be used as an permeation enhancer.

Among these permeation enhancers, preferable examples include caprylic acid, capric acid, caproic acid, lauric

acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methyl laurate, hexyl laurate, lauric acid diethanol amide, isopropyl myristate, myristyl myristate, octyldodecyl myristate, cetyl palmitate, salicylic acid, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, L-menthol, borneol, d-limonene, isoeugenol, isoborneol, nerol, dl-camphor, glycerin monocaprirate, glycerin monocaprate, glycerin monolaurate, glycerin monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pirotiodecane and olive oil. Among them, pirotiodecane, isopropyl myristate, lauryl alcohol, myristyl alcohol, isostearyl alcohol, lauric acid diethanol amide, glycerin monocaprirate, glycerin monocaprate, glycerin monooleate, sorbitan monolaurate, propylene glycol monolaurate and polyoxyethylene lauryl ether are more preferable, and pirotiodecane, and/or isopropyl myristate are particularly preferable. Such permeation enhancers may be used alone, or may be used in combination of two or more kinds.

Compounding amount of the permeation enhancer according to the present invention is not particularly limited, but it is preferably 0.1-20 % by weight, more preferably 0.1-10 % by weight on the basis of the total amount of the compounds contained in the adhesive layer. A compounding amount of the permeation enhancer lower than the lowest limit described above tends to give an insufficient effect on enhancing skin permeability of the drug provided by compounding the permeation enhancer and, on the other hand, that higher than the highest limit described above tends to increase skin irritating property.

In the patch for transdermal administration of the present invention, the adhesive layer may comprise additionally cyclodextrin and/or a derivative thereof as a permeation enhancing auxiliary agent. Examples of cyclodextrin or the derivative thereof according to the present invention include α, β or γ-cyclodextrin, dimethyl-(α, β or γ) -cyclodextrin, diethyl-(α, β or γ)-cyclodextrin, triethyl-(α, β or γ)-cyclodextrin, carboxymethylethyl-(α, β or γ)-cyclodextrin, carboxymethyl-(α, β or γ)-cyclodextrin, 2,6-dimethyl-O-methyl-(α, β or γ)-cyclodextrin, 2,3,6-trimethyl-O-methyl- (α, β or γ) -cyclodextrin, 2-hydroxyethyl- (α, β or γ)-cyclodextrin, 2-hydroxypropyl-(α, β or γ)-cyclodextrin, 3-hydroxypropyl-(α, β or γ)-cyclodextrin, 2,3-dihydroxypropyl- (α, β or γ)-cyclodextrin, which are utilized in the field of food or pharmaceutical product. Branched cyclodextrin derivatives and cyclodextrin polymer derivatives may also be included.

As such cyclodextrin or derivatives thereof, those with high water solubility are preferable and, among them, hydrophilic cyclodextrin such as 2,6-dimethyl-O-methyl- (α, β or γ) -cyclodextrin, 2,3,6-rimethyl-O-methyl-(α, β or γ)-cyclodextrin, 2-hydroxyethyl- (α, β or γ) -cyclodextrin, 2-hydroxypropyl-(α, β or γ)-cyclodextrin, 3-hydroxypropyl-(α, β or γ)-cyclodextrin and 2,3-dihydroxypropyl-(α, β or γ)-cyclodextrin are more preferable, and 2-hydroxyethyl-(α, β or γ)-cyclodextrin is especially preferable. These cyclodextrin or derivatives thereof may be used alone or in combination of two or more kinds.

Compounding amount of the cyclodextrin or the derivative thereof according to the present invention is not particularly limited, but a compounding ratio to the drug [drug:(cyclodextrin or derivative thereof)] is preferably from 1:0.05 to 1:20 (mol ratio), more preferably from 1:0.1 to 1:10 (mol ratio). A compounding amount of the cyclodextrin lower than the lowest limit described above tends to give an insufficient effect on enhancing skin permeability of the medical agent and, on the other hand, that higher than the highest limit described above tends to decrease the cohesion force.

In the patch for transdermal administration of the present invention, the adhesive layer may further comprise a plasticizer. Specific examples of the plasticizer according to the present invention include petroleum oil (paraffin process oil, naphthene process oil, aromatic process oil and the like), squalane, squalene, vegetable oil (olive oil, camellia oil, castor oil, tall oil, peanut oil and the like), silicone oil, dibasic acid ester (dibutyl phthalate, dioctyl phthalate and the like), liquid rubber (polybutene, liquid isoprene rubber and the like), liquid fatty acid ester (isopropyl myristate, hexyl laurate, diethyl sebacate, diisopropyl sebacate and the like), polyhydric alcohol (diethylene glycol, polyethylene glycol, glycol salicylate, propylene glycol, dipropylene glycol and the like), triacetin, triethyl citrate, crotamiton and the like. Among these plasticizers, polyhydric alcohols, liquid paraffin, liquid polybutene, crotamiton, diethyl sebacate and hexyl laurate are more preferable, and polyethylene glycol is especially preferable. These plasticizers may be used alone or in combination of two or more kinds.

Compounding amount of the plasticizer according to the present invention is not particularly limited, but it is preferably 5-70 % by weight, more preferably 10-60 % by weight on the basis of the total amount of the compounds contained in the adhesive layer. A compounding amount of the plasticizer lower than the lower limit described above tends to give an insufficient effect on enhancing a cohesion force of the patch provided by compounding a plasticizer and, on the other hand, that higher than the highest limit described above tends to give an insufficient skin permeability of the drug.

In the patch for transdermal administration of the present invention, the adhesive layer may further comprise a tackifying resin. Specific examples of the tackifying resin according to the present invention include rosin derivatives (rosin, rosin glycerine ester, hydrogenated rosin, hydrogenated rosin glycerine ester, rosin pentaerythritol ester and the like), alicyclic saturated hydrocarbon resin (Arkon P100 (manufactured by Arakawa Chemical Industries) and the like),

aliphatic hydrocarbon resin (Quintone B-170 (manufactured by ZEON CORPORATION) and the like), terpene resin (Clearon P-125 (manufactured by Yasuhara Chemical) and the like), maleic acid resin and the like. Among them, hydrogenated rosin glycerine ester, alicyclic saturated hydrocarbon resin, aliphatic hydrocarbon resin and terpene resin are more preferable, and alicyclic saturated hydrocarbon resin is especially preferable.

Compounding amount of the tackifying resin according to the present invention is not particularly limited, but it is preferably 5-70 % by weight, more preferably 5-60 % by weight on the basis of the total amount of the compounds contained in the adhesive layer. A compounding amount of the tackifying resin lower than the lowest limit described above tends to give an insufficient effect on enhancing adhesibility of the patch provided by compounding the tackifying resin and, on the other hand, that higher than the highest limit described above tends to increase skin irritating property upon peeling the patch.

In the patch for transdermal administration of the present invention, the adhesive layer may further comprise organic acids. Examples of such organic acid include aliphatic (mono-, di-, tri-) carboxylic acids (acetic acid, propionic acid, citric acid, isobutyric acid, caproic acid, caprylic acid, lactic acid, maleic acid, pyruvic acid, oxalic acid, succinic acid, tartaric acid and the like), aromatic carboxylic acids (phthalic acid, salicylic acid, benzoic acid, acetyl salicylic acid and the like), alkyl sulfonic acids (methanesulfonic acid, ethanesulfonic acid, propylsulfonic acid, butanesulfonic acid, polyoxyethylenealkyl ether sulfonic acid and the like), alkyl sulfonic acid derivatives (N-2-hydroxyethylpiperidine-N'-2-ethanesulfonic acid), cholic acid derivatives (dehydrocholic acid and the like) and salts thereof. Among these organic acids, monocarboxylic acids or alkyl sulfonic acids are preferable, and acetic acid is especially preferable. These organic acids may be used alone or in combination of two or more kinds.

Compounding amount of the organic acid according to the present invention is not particularly limited, but it is preferably 0.01-20 % by weight, more preferably 0.1-15 % by weight on the basis of the total amount of the compounds contained in the adhesive layer. A compounding amount of the organic acid lower than the lowest limit described above tends to give an insufficient effect on enhancing skin permeability of the drug provided by compounding the organic acid and, on the other hand, that higher than the highest limit described above tends to increase skin irritating property.

Further, in the present invention, the adhesive layer may be compounded with an antioxidant, a filler, a crosslinker, a preservative, an ultraviolet absorbent or the like if necessary. Preferable examples of the antioxidant according to the present invention include tocopherols and ester derivatives thereof, ascorbic acid, ascorbyl stearate, nordihydroguaiaretic acid, dibutylhydroxytoluene (BHT) and butylhydroxyanisol.

Preferable examples of the filler include calcium carbonate, magnesium carbonate, silicate (such as aluminum silicate and magnesium silicate), silicic acid, barium sulfate, calcium sulfate, calcium zincate, zinc oxide and titanium oxide.

Preferable examples of the crosslinker include a thermosetting resin such as amino resin, phenol resin, epoxy resin, alkyd resin and unsaturated polyester, an isocyanate compound, a block isocyanate compound, an organic crosslinker, and an inorganic crosslinker such as metal and metal compounds.

Preferable examples of the preservative include ethyl parahydroxybenzoate, propyl parahydroxybenzoate and butyl parahydroxybenzoate.

Preferable examples of the ultraviolet absorbent include p-aminobenzoic acid derivatives, anthranilic acid derivatives, salicylic acid derivatives, coumarin derivatives, amino acid-series compounds, imidazoline derivatives, pyrimidine derivatives and dioxane derivatives.

Respective compounding amounts of the antioxidant, the filler, the crosslinker, the preservative and the ultraviolet absorbent are not particularly limited, but the total amount of the antioxidant, the filler, the crosslinker, the preservative and the ultraviolet absorbent is preferably 0-10 % by weight, more preferably 0-5 % by weight on the basis of the total amount of the compounds contained in the adhesive layer.

The patch for transdermal administration of the present invention is a patch in which the adhesive layer described above is placed on at least one side of the backing described above, and the method of placing the adhesive layer on the backing is not particularly limited. For example, a mixture of the adhesive base agent, the drug, and other ingredients described above that are added according to need is thermally molten and coated on the backing to provide the patch of the present invention. Further, when the patch of the present invention further comprises release liner on the adhesive layer, the thermally molten mixture described above is coated on the release liner followed by laying the backing on the coated side, or the thermally molten mixture described above is coated on the backing followed by laying the release liner on the coated side to provide the patch of the present invention. Furthermore, instead of thermally melting the mixture described above, it is also possible to use a coating liquid prepared by dissolving the above mentioned mixture in a solvent such as toluene, hexane or ethyl acetate to provide the patch of the present invention.

Here, the patch of the present invention may be a patch provided with one adhesive layer, or a patch provided with two or more adhesive layers insofar as they do not degrade skin permeability of the drug.

Further, film thickness of the adhesive layer according to the present invention is not particularly limited, but it is preferably 20-200 μm. A film thickness of the adhesive layer lower than the lowest limit described above tends to give an insufficient skin permeability of the drug and, on the other hand, that higher than the highest limit described above tends to generate a phenomenon in which the adhesive agent remains adhering to the skin after application (adhesive

agent remaining).

Furthermore, when the patch of the present invention comprises release liner, specific examples of such release liner include film made of polyester such as polyethylene terephthalate, polyvinyl chloride, polyvinylidene chloride or the like, and laminate film of fine paper and polyolefin. In these release liners, it is preferable to provide silicone treatment to the side contacting the adhesive layer, because operation easiness upon peeling the release liner off the patch is enhanced.

## Examples

The present invention will be explained further specifically on the basis of Examples and Preliminary Tests, however the present invention is not limited to these examples. In the following Examples and Preliminary Test, "%" means "% by weight" if not otherwise specified.

Preliminary Test 1 (permeability test through hairless mouse skin)

A permeability test through hairless mouse skin was conducted for the following 1-12 test solutions:

Test solution 1: a 1 % (w/v) solution of valdecoxib in acetone,
Test solution 2: a saturated solution of valdecoxib in liquid paraffin,
Test solution 3: a saturated solution of valdecoxib in propylene glycol (PG),
Test solution 4: a saturated solution of valdecoxib in isopropyl myristate (IPM),
Test solution 5: a 1% (w/v) solution of celecoxib in acetone,
Test solution 6: a saturated solution of celecoxib in liquid paraffin,
Test solution 7: a saturated solution of celecoxib in propylene glycol (PG),
Test solution 8: a saturated solution of celecoxib in isopropyl myristate (IPM),
Test solution 9: a 1% (w/v) solution of rofecoxib in acetone,
Test solution 10: a saturated solution of rofecoxib in liquid paraffin,
Test solution 11: a saturated solution of rofecoxib in propylene glycol (PG), and
Test solution 12: a saturated solution of rofecoxib in isopropyl myristate (IPM).

First, dorsal skin of a hairless mouse was extirpated, which was set to a flow-through cell (effective area: 1 $cm^2$) with facing the dermis side to the receptor side, where a water of 37 °C was circulating the periphery of the cell. Next, 1 mL of Test solution 1 was charged in a cell on the stratum corneum side of the skin as a donor solution. While using a mixed solution of 40 % (v/v) polyethylene glycol 400/physiological saline as a receptor layer, sampling of the receptor solution was carried out at a speed of 5 mL/hour every 2 hours up to 24 hours. For receptor solutions obtained at respective times, the flow volume was measured precisely and the drug concentration was measured by using high-performance liquid chromatography. From the obtained measurement values of the flow volume and drug concentration, the permeability rate per 1 hour was calculated to obtain the maximum value of the permeability rate of the drug per unit area of the skin. The obtained results are listed in Table 1.

Table 1

| Base agent<br>Medical agent | Acetone ($\mu g/cm^2/hr$) | Liquid paraffin ($\mu g/cm^2/hr$) | PG ($\mu g/cm^2/hr$) | IPM ($\mu g/cm^2/hr$) |
|---|---|---|---|---|
| valdecoxib | 0.59 | 0.41 | 4.10 | 2.97 |
| celecoxib | 0.3 | 0.00 | 0.90 | 2.04 |
| rofecoxib | 0.09 | 0.00 | 0.00 | 0.00 |

As is evident from the results listed in Table 1, it was confirmed that valdecoxib has a very high skin permeability compared with celecoxib and rofecoxib.

Preliminary Test 2 (yeast-induced paw inflammation and nociception test in rats)

First, SD male rats with weight of around 130 g were used to measure a pain threshold value (g) by using analgesimeter (Analgesy 7200, Ugo Basile) according to the method of Randall & Selitto [Rahdall, L. O. and Selitto, J. J. A method for measurement of analgesic activity on inflamed tissue. (1957) Arch. Int. Pharmacodyn. 4, 409-419], while defining removal reaction against pressure irritation to a right hind footpad as the index, and they were grouped such

that there was no difference between the average pain threshold values of both groups.

Next, each 100 μL of a 3 % (w/v) solution of valdecoxib in acetone was applied to the right hind footpad. On the other hand, acetone was applied to the control group. After sufficient drying after the application, the rats were housed in separate cages without covering treatment. At this time, for the purpose of preventing oral drug intake, a collar was attached to all the rats including the control group. After 3 hours from the application described above, valdecoxib remaining at the applied site was wiped off with absorbent cotton having been dipped in slightly warm water and, immediately after the wipe, each 100 μL of a yeast suspension (20 % Baker's Yeast) was injected subcutaneously to all the rats including the control group to induce inflammation. After additional 3 hours, the pain threshold value at the right hind footpad was measured again to evaluate the analgesic effect of valdecoxib. The obtained results are illustrated in Fig. 1. In this connection, data in Fig. 1 show an average value ± standard error, and ** means "significantly different (Student's t-test)" with p<0.01.

As is evident from the results illustrated in Fig. 1, it was confirmed that valdecoxib exerts a significant analgesic action as a specific cyclooxygenase-2 inhibitor (COX-2 inhibitor).

Preliminary Test 3 (carrageenin-induced paw edema test in rats)

First, SD male rats with weight of around 110 g were used to measure a paw volume (mL) of a right hind footpad by using a water-replacement type paw volume determining device (Model TK-101 CMP, MUROMACHI KIKAI) according to the method of Fujihira et al. [Fujihira Eichi, Screening no Hoho to Mondaiten - Adjuvant Kansetsuen ni yoru Koenshoyaku no Kenteiho (Methods and Problems of Screening - Assay of Antiinflammatory Agent by Adjuvant Arthritis) (1971) Oyo Yakuri (Pharmacometrics) 5,169-183], and they were grouped such that there was no difference between the average paw volume values of both groups. Next, each 100 μL of a 3 % (w/v) solution of valdecoxib in acetone was applied to the right hind footpad. On the other hand, acetone was applied to the control group. After sufficient drying after the application, the rats were housed in separate cages without covering treatment. At this time, for the purpose of preventing oral drug intake, a collar was attached to all the rats including the control group. After 3 hours from the application described above, valdecoxib remaining at the applied site was wiped with absorbent cotton having been dipped in slightly warm water and, immediately after the wipe, each 100 μL of carrageenin (1 % λ carrageenin) was injected subcutaneously to all the rats including the control group to induce inflammation. After additional 3 hours, the paw volume at the right hind footpad was measured again to calculate an edema ratio (%) according to the following edema ratio calculation method (calculation formula):

Edema ratio (%) = [(paw volume after 3 hours from inflammation (mL) - paw volume at ordinary time (mL))/ paw volume at ordinary time (mL))] × 100

to evaluate the antiinflammatory effect of valdecoxib. The obtained results are illustrated in Fig. 2. In this connection, data in Fig. 2 show an average value ± standard error, and ** means "significantly different (Student's t-test)" with p<0.01.

As is evident from the results illustrated in Fig. 2, it was confirmed that valdecoxib exerts a significant antiinflammatory action as a specific COX-2 inhibitor.

Example 1 (Preparation of a patch)

Valdecoxib, pirotiodecane and isopropyl myristate were charged in a mortar and mixed sufficiently, then the obtained mixture was added to a mixed solution consisting of an acrylic polymer (DURO-TAK87-4098, manufactured by National Starch & Chemical) and ethyl acetate to prepare a coating liquid (Vx-007 formulation) having the following composition (excluding solvent).

| | |
|---|---|
| Acrylic polymer (Duro Tak87-4098) | 64.3% |
| Isopropyl myristate (IPM) | 25.7% |
| Pirotiodecane | 5.0% |
| Valdecoxib | 5.0% |
| Total | 100.0% |

Next, the obtained coating liquid was applied to a release liner made of polyethylene terephthalate and dried to remove the solvent to form an adhesive layer (thickness: 100 μm). Then, on the adhesive layer, a backing made of polyethylene terephthalate was laminated to provide a targeted patch for transdermal administration (acrylic tape agent).

(Carrageenin-induced paw edema test in rats)

First, grouping was conducted in the same way as Preliminary Test 3 except that SD male rats with weight of around 130 g were used. Next, the acrylic patch containing 5 % valdecoxib obtained as described above (3 cm × 3 cm) was adhered so as to wrap around a right hind footpad. On the other hand, a placebo group was stuck with cellophane tape so as to wrap around a right hind footpad. Then, after attaching a collar to all the rats including the control group for the purpose of preventing oral drug intake, the rats were housed in separate cages. After 3 hours from the sticking, the patch and cellophane tape were peeled off and, immediately after the peeling, each 100 μL of carrageenin (1 % λ carrageenin) was injected subcutaneously to all the rats including the control group to induce inflammation. After additional 3 hours, the paw volume at the right hind footpad was measured again to calculate the edema ratio (%) in the same way as Preliminary Test 3 to evaluate the antiinflammatory effect of the patch containing valdecoxib. The obtained results are illustrated in Fig. 3. In this connection, data in Fig. 3 show an average value ± standard error, and ** means "significantly different (Student's t-test)" with $p<0.01$.

As is evident from the results illustrated in Fig. 3, it was confirmed that a significant antiinflammatory action is exerted by the patch for transdermal administration (acrylic tape agent) of the present invention containing valdecoxib which is a COX-2 inhibitor.

Example 2 (Preparation of a patch)

Valdecoxib, pirotiodecane, β-cyclodextrin, polyethylene glycol 400 (Macrogol 400, manufactured by NOF CORPORATION) and liquid paraffin were charged in a mortar and mixed sufficiently. The obtained mixture was added to a mixed solution consisting of styrene-isoprene-styrene block copolymer, polyisobutylene, alicyclic saturated hydrocarbon resin (Arkon P100, manufactured by Arakawa Chemical Industries), ethyl acetate and toluene to prepare a coating liquid (Vx-009 formulation) having the following composition (excluding the solvent).

| | |
|---|---|
| SIS | 15.2% |
| PIB | 6.5% |
| Alicyclic saturated hydrocarbon resin (Arkon P100) | 39.1% |
| Liquid paraffin | 21.7% |
| β-cyclodextrin | 4.5% |
| Polyethylene glycol 400 | 5.0% |
| Pirotiodecane | 3.0% |
| Valdecoxib | 5.0% |
| Total | 100.0% |

Next, the obtained coating liquid was applied to a release liner made of polyethylene terephthalate and dried to remove the solvent to form an adhesive layer (thickness: 100 μm). Then, on the adhesive layer, a backing made of polyethylene terephthalate was laminated to provide a targeted patch for transdermal administration (SIS-based tape agent).

(Carrageenin-induced paw edema test in rats)

The edema ratio (%) was calculated in the same way as Example 1 except for using SD male rats with weight of around 145 g, using the SIS-based patch (3 cm × 3 cm) containing 5 % of valdecoxib obtained as described above, and peeling the patch and cellophane tape after 5 hours from the sticking, to evaluate the antiinflammatory effect of the patch containing valdecoxib. The obtained results are illustrated in Fig. 4. In this connection, data in Fig. 4 show an average value ± standard error, and ** means "significantly different (Student's t-test)" with $p<0.01$.

As is evident from the results illustrated in Fig. 4, it was confirmed that a significant antiinflammatory action is also exerted by the patch for transdermal administration (SIS-based tape agent) of the present invention containing valdecoxib which is a COX-2 inhibitor.

## Industrial Applicability

As explained above, according to the present invention, it was found that valdecoxib has a sufficiently high transdermal permeation property and that, by using such valdecoxib or a pharmaceutically acceptable salt thereof, it is possible to obtain a patch for transdermal administration having a sufficiently high transdermal permeation property of a COX-2 inhibitor and exerting a superior antiinflammatory effect.

## Claims

1. A patch for transdermal administration comprising a backing and an adhesive layer which is placed on at least one side of the backing and comprises an adhesive base agent and a drug, wherein the drug is valdecoxib or a pharmaceutically acceptable salt thereof.

2. The patch for transdermal administration according to claim 1, wherein the adhesive base agent comprises at least one kind selected from the group consisting of styrene-isoprene-styrene copolymer, acrylic polymer and polyisobutylene.

3. The patch for transdermal administration according to claim 2, wherein the adhesive base agent comprises styrene-isoprene-styrene copolymer and polyisobutylene.

4. The patch for transdermal administration according to any one of claims 1 to 3, wherein the adhesive layer further comprises a polyhydric alcohol.

5. The patch for transdermal administration according to claim 4, wherein the polyhydric alcohol is polyethylene glycol.

6. The patch for transdermal administration according to claim 4, wherein the adhesive layer further comprises cyclodextrin and/or a derivative thereof.

7. The patch for transdermal administration according to claim 5, wherein the adhesive layer further comprises cyclodextrin and/or a derivative thereof.

8. The patch for transdermal administration according to claim 4, wherein the adhesive layer further comprises pirotiodecane.

9. The patch for transdermal administration according to claim 5, wherein the adhesive layer further comprises pirotiodecane.

10. The patch for transdermal administration according to claim 6, wherein the adhesive layer further comprises pirotiodecane.

11. The patch for transdermal administration according to claim 7, wherein the adhesive layer further comprises pirotiodecane.

## Patentansprüche

1. Ein Pflaster zur transdermalen Verabreichung, umfassend einen Träger und eine Klebstoffschicht, die auf mindestens einer Seite des Trägers angeordnet ist und eine Klebstoffbasis und einen Wirkstoff umfasst, **gekennzeichnet dadurch, dass** der Wirkstoff Valdecoxib oder ein pharmakologisch verträgliches Salz davon ist.

2. Das Pflaster zur transdermalen Verabreichung nach Anspruch 1, wobei die Klebstoffbasis mindestens eines ausgewählt aus der Gruppe bestehend aus Styrol-Isopren-Styrol-Copolymer, Acrylpolymer und Polyisobutylen umfasst.

3. Das Pflaster zur transdermalen Verabreichung nach Anspruch 2, wobei die Klebstoffbasis Styrol-Isopren-Styrol-Copolymer und Polyisobutylen umfasst.

4. Das Pflaster zur transdermalen Verabreichung nach einem der Ansprüche 1 bis 3, wobei die Klebstoffschicht ferner

einen mehrwertigen Alkohol umfasst.

5. Das Pflaster zur transdermalen Verabreichung nach Anspruch 4, wobei der mehrwertige Alkohol Polyethylenglykol ist.

6. Das Pflaster zur transdermalen Verabreichung nach Anspruch 4, wobei die Klebstoffschicht ferner Cyclodextrin und/oder ein Derivat davon umfasst.

7. Das Pflaster zur transdermalen Verabreichung nach Anspruch 5, wobei die Klebstoffschicht ferner Cyclodextrin und/oder ein Derivat davon umfasst.

8. Das Pflaster zur transdermalen Verabreichung nach Anspruch 4, wobei die Klebstoffschicht ferner Pirotiodecan umfasst.

9. Das Pflaster zur transdermalen Verabreichung nach Anspruch 5, wobei die Klebstoffschicht ferner Pirotiodecan umfasst.

10. Das Pflaster zur transdermalen Verabreichung nach Anspruch 6, wobei die Klebstoffschicht ferner Pirotiodecan umfasst.

11. Das Pflaster zur transdermalen Verabreichung nach Anspruch 7, wobei die Klebstoffschicht weiterhin Pirotiodecan umfasst.

## Revendications

1. Timbre destiné à une administration transdermique comprenant un support et une couche adhésive qui est placée sur au moins un côté du support et comprend un agent de base adhésif et un médicament, dans lequel le médicament est le valdécoxib ou un sel pharmaceutiquement acceptable de celui-ci.

2. Timbre destiné à une administration transdermique selon la revendication 1, dans lequel l'agent de base adhésif comprend au moins un composant sélectionné parmi le groupe constitué par un copolymère styrène-isoprène-styrène, un polymère acrylique et du polyisobutylène.

3. Timbre destiné à une administration transdermique selon la revendication 2, dans lequel l'agent de base adhésif comprend un copolymère styrène-isoprène-styrène et du polyisobutylène.

4. Timbre destiné à une administration transdermique selon l'une quelconque des revendications 1 à 3, dans lequel la couche adhésive comprend en outre un alcool polyhydrique.

5. Timbre destiné à une administration transdermique selon la revendication 4, dans lequel l'alcool polyhydrique est le polyéthylène glycol.

6. Timbre destiné à une administration transdermique selon la revendication 4, dans lequel la couche adhésive comprend en outre de la cyclodextrine et/ou un dérivé de celle-ci.

7. Timbre destiné à une administration transdermique selon la revendication 5, dans lequel la couche adhésive comprend en outre de la cyclodextrine et/ou un dérivé de celle-ci.

8. Timbre destiné à une administration transdermique selon la revendication 4, dans lequel la couche adhésive comprend en outre du pirotiodécane.

9. Timbre destiné à une administration transdermique selon la revendication 5, dans lequel la couche adhésive comprend en outre du pirotiodécane.

10. Timbre destiné à une administration transdermique selon la revendication 6, dans lequel la couche adhésive comprend en outre du pirotiodécane.

**11.** Timbre destiné à une administration transdermique selon la revendication 7, dans lequel la couche adhésive comprend en outre du pirotiodécane.

*Fig.1*

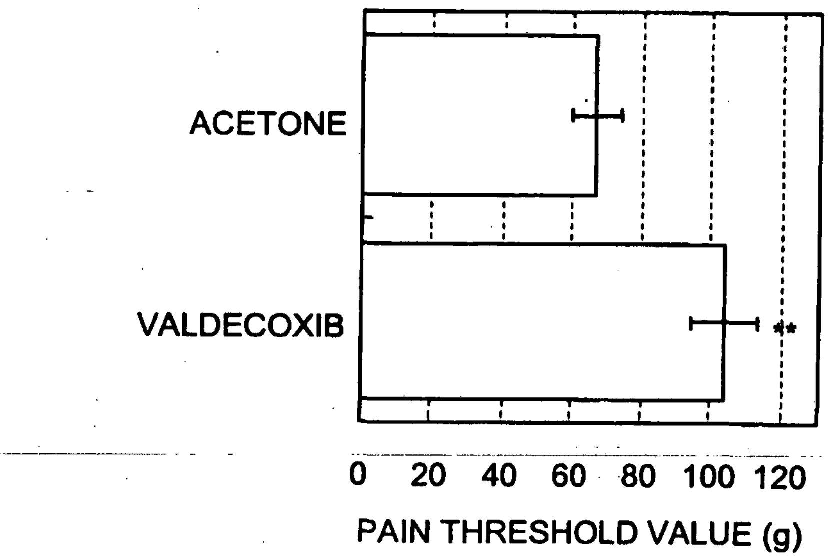
ACETONE
VALDECOXIB
**
0 20 40 60 80 100 120
PAIN THRESHOLD VALUE (g)

*Fig.2*

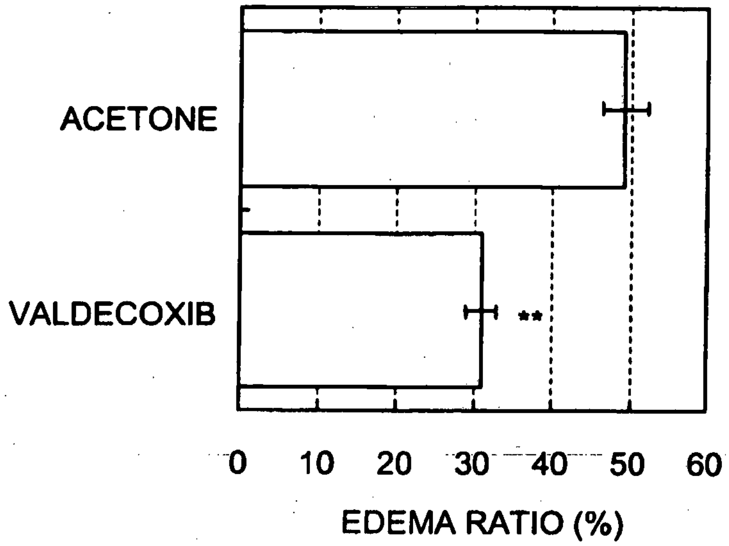

*Fig.3*

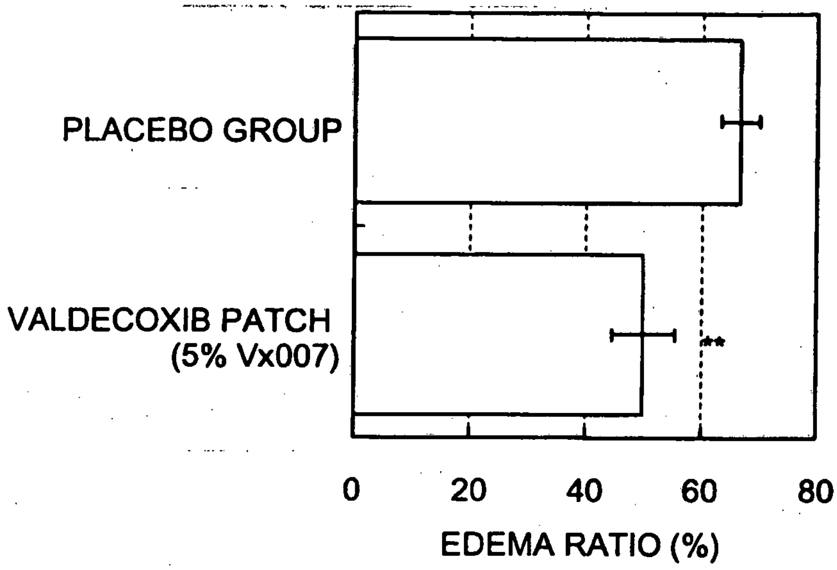
PLACEBO GROUP
VALDECOXIB PATCH
(5% Vx007)
**
0
20
40
60
80
EDEMA RATIO (%)

Fig.4

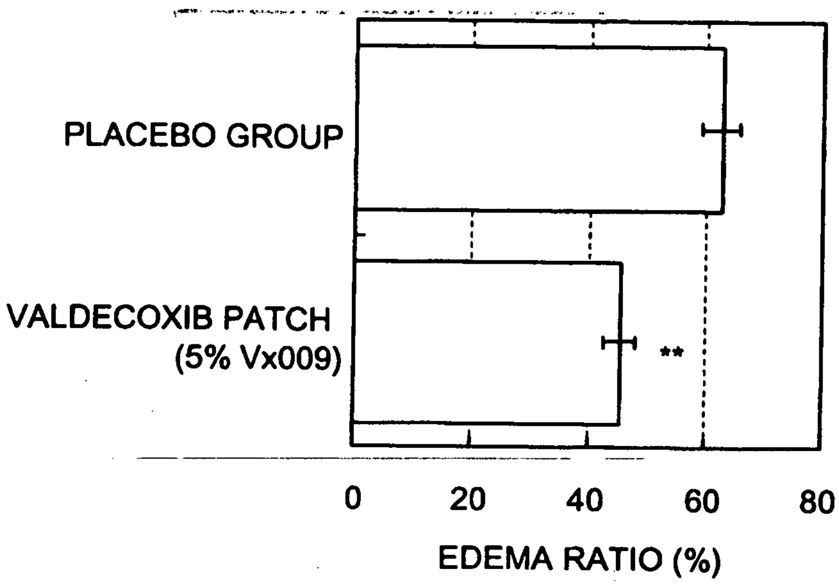
PLACEBO GROUP
VALDECOXIB PATCH
(5% Vx009)
**
0
20
40
60
80
EDEMA RATIO (%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0202111 A


### Non-patent literature cited in the description


- **J. J. TALLEY.** 4-[5-Methyl-3-phenylisoxazol-4-yl]-benzenesulfonamide, Valdecoxib: A Potent and Selective Inhibitor of COX-2. *J. Med. Chem.,* 2000, vol. 43, 775-777
- Iyakuhin Tenkabutsu Jiten. 2000
- **RAHDALL, L. O. ; SELITTO, J. J.** A method for measurement of analgesic activity on inflamed tissue. *Arch. Int. Pharmacodyn.,* 1957, vol. 4, 409-419
- **FUJIHIRA EICHI.** Screening no Hoho to Mondaiten - Adjuvant Kansetsuen ni yoru Koenshoyaku no Kenteiho. *Oyo Yakuri,* 1971, vol. 5, 169-183